# EUROPÄISCHE PATENTSCHRIFT

(11) **EP 1 810 636 B1**
(45) Veröffentlichungstag und Bekanntmachung des Hinweises auf die Patenterteilung: **06.01.2016**
(21) Anmeldenummer: 06001408.1
(22) Anmeldetag: 24.01.2006
(51) Int. Cl.: A61B 19/08

(54) **Sterile Abdeckung**
Sterile cover
Élément couvrant stérile

(43) Veröffentlichungstag der Anmeldung: 25.07.2007
(73) Patentinhaber: Brainlab AG, 85622 Feldkirchen (DE)
(72) Erfinder: Bogojevic, Aleksander, 80797 München (DE); Maier, Christian, 80802 München (DE); Maier, Georg, 81541 München (DE); Uhde, Jörg, 80538 München (DE)
(74) Vertreter: Schwabe - Sandmair - Marx

(56) Entgegenhaltungen:
- US-A- 5 490 524
- US-A- 5 812 188
- US-A- 5 891 020
- US-A1- 2001 036 245
- US-A1- 2003 153 810

## Beschreibung

Die Erfindung betrifft eine sterile Abdeckung, wie sie verwendet wird, um Gegenstände in einem sterilen Umfeld abzudecken, beispielsweise in Operationssälen. Insbesondere eignet sich die sterile Abdeckung für Trackingaufsätze von C-Bogen-Röntgengeräten.

C-Bögen bzw. deren Trackingaufsätze werden herkömmlicherweise mit schlauch- oder beutelförmigen Folien steril abgedeckt. Es gibt eine Anzahl von Abdeckungen für C-Bögen, die zum sterilen Abdecken des Bildverstärkers (oder anderer Teile) eingesetzt werden. Diese Abdeckungen haben die gemeinsame Eigenschaft, dass sie eine Innenseite aufweisen, welche dem abzudeckenden Teil (C-Bogen oder dessen Teile z.B. zylinderförmiger Trackingaufsatz mit Reflektoren-Markeraufsätzen) zugewandt sind und eine äußere sterile Seite, welche der sterilen Umgebung des Operationsraumes zugewandt ist.

Herkömmliche C-Bogen-Abdeckungen sind beispielsweise aus der US 6,697,664 und aus der US 5,802,719 bekannt, wobei das letztere Patent eine sterile Abdeckung zum Abdecken des inneren Teils des C-Bogens umfasst. Diese Abdeckung ist flexibel und wird mit einer Anzahl von Clips in Form gezogen, die an dem Rahmen des C-Bogens zu befestigen sind. Aus der US 6,481,888 ist eine Abdeckung zum Abschirmen von Röntgenstrahlen bekannt, die vom OP-Tisch gestreut werden.

Die bekannten Abdeckungsfolien sind aus einem relativ dünnen Material hergestellt; dies ist zunächst auch so gewollt, damit die Abdeckungen flexibel bleiben und gut über die Gegenstände drapiert werden können. Die herkömmlichen Abdeckungen sind aber derart flexibel, dass sie Falten und Überwürfe bilden, wenn sie über einen Gegenstand gezogen werden, und solche Falten und Überwürfe werden dann zum Problem, wenn der abzudeckende Gegenstand durch ein Trackingsystem erfasst werden soll. Optische Trackingsysteme empfangen entweder aktiv abgestrahlte, meist im Infrarotbereich leuchtende Lichtblitze oder Lichtreflexionen, die beispielsweise von speziellen Reflektorenmarkern abgegeben werden. Trackingaufsätze für die Bildverstärker von C-Bögen haben z.B. eine Anzahl solcher Reflektorenmarker auf ihrer äußeren, am Umfang zylindrischen Oberfläche. Wenn diese Marker nun mit einer Folie bedeckt sind, die Falten und Überwürfe hat, und wenn beispielsweise eine Falte über einem Reflektionsmarker liegt, kann dessen Reflektionsbild verfälscht (gestreut) werden. Dies kann dazu führen, dass der Marker von einem Kamera-Trackingsystem an einer falschen Stelle geortet wird, was wiederum die Registrierung der mit dem C-Bogen erzeugten Röntgenaufnahmen verfälscht.

Das Dokument US 2001/036245 A, das die Basis des Oberbegriffs von Anspruch 1 formt, offenbart ein Steriles Abdecksystem zum terilen Verkleidung eines Trackingaufsatzes von C-Bogen-Rötgengeräten mit einer durchsichtigen Folie.

Eine weitere Vorrichtung zum sterilen Verkleidung eines Trackingaufsatzes von C-Bogen-Rötgengeräten ist aus der US 5490524 A bekannt.

Es ist die Aufgabe der vorliegenden Erfindung, eine sterile Abdeckung bereitzustellen, welche die oben aufgeführten Nachteile des Standes der Technik überwindet. Insbesondere soll die eindeutige Sichtbarkeit und/oder Ortsbestimmbarkeit von abgedeckten Gegenständen gewährleistet sein.

Diese Aufgabe wird erfindungsgemäß durch eine sterile Abdeckung gemäß dem Anspruch 1 gelöst. Die Unteransprüche definieren bevorzugte Ausführungsformen der Erfindung.

Die erfindungsgemäße sterile Abdeckung weist einen verstärkten Folienabschnitt auf, sowie eine Halte- bzw. Spanneinrichtung mittels welcher der verstärkte Folienabschnitt an oder über einer Oberfläche straff- bzw. glatt gezogen werden kann. Mit anderen Worten wird zumindest ein Teil der Abdeckung durch die erfindungsgemäße Materialausgestaltung faltenfrei gemacht, und es wird dafür gesorgt, dass zumindest dieser faltenlose und überwurfslose, verstärkte Folienabschnitt eng an dem abzudeckenden Gegenstand anliegen kann, nämlich durch die Halte- bzw. Spanneinrichtung. Auf diese Weise kann nun sichergestellt werden, dass der abgedeckte Gegenstand oder zumindest seine wichtigen Teile von einem glatten (und insbesondere auch straff gezogenen) eng anliegenden Folienteil abgedeckt sind, so dass erkennenswerte Gegenstände auf der Oberfläche auch durch die Abdeckung hindurch erkannt werden können.

Die erfindungsgemäße sterile Abdeckung umfasst mit anderen Worten einen dimensionsstabilen oder abmessungsstabilen bzw. formstabilen Folienabschnitt, der z.B. verwendet werden kann, um einen Teil eines C-Bogens abzudecken, der für ein Trackingsystem sichtbar sein muss. Dieser Teil des C-Bogens kann das Registrierungs-Kit (Trackingaufsatz) sein, das bei der bildgeführten Chirurgie verwendet wird. Das Registrierungs-Kit umfasst Marker, die optisch durch ein Kamerasystem getrackt werden. Das bildgeführte Navigationssystem kann mit den Markern, die wegen der erfindungsgemäß ausgebildeten sterilen Abdeckung immer gut und eindeutig sichtbar sind, genau arbeiten, weil das Trackingsystem nur bei eindeutig sichtbaren Markern genaue Markerpositionen liefert.

Gemäß einer Ausführungsform ist die sterile Abdeckung bzw. zumindest ihr verstärkter Abschnitt als Umgriff oder Umhüllung für den abzudeckenden Gegenstand ausgebildet. Sie kann als beutelartige Umhüllung mit einem verstärkten Abschnitt und einem nicht verstärkten Restabschnitt ausgebildet sein. Als Umgriff oder Umhüllung eignet sich die erfindungsgemäße Abdeckung speziell auch für glatte bzw. ebene Flächen, d.h. für alle Geräte, deren Oberflächenmerkmale gut sichtbar sein sollen und auf denen die Folie flach aufliegen soll. Ein Beispiel ist die zylinderartige Außenfläche eines oben genannten Registrierungs-Kitts.

Der verstärkte Folienabschnitt kann erfindungsgemäß formverstärkt sein, so dass er insbesondere eine glatte Form aufrechterhalten kann. Gemäß einer Ausgestaltung kann er ein Material mit den folgenden Eigenschaften aufweisen:
- einer mehr als 1,5-fachen, insbesondere mehr als 3-4 fachen, speziell mehr als 6 bis 8-fachen Dicke einer klinischen, sterilen Abdeckfolie; bzw.
- einer 1,5 bis 15-fachen, insbesondere 4 bis 12-fachen, speziell 7 bis 10-fachen Dicke einer klinischen, sterilen Abdeckfolie (die z.B. 30 bis 50 µm dick sein kann).

Ferner kann der verstärkte Folienabschnitt ein Material mit den folgenden Eigenschaften aufweisen:
- einer mehr als 1,5-fachen, insbesondere mehr als 3-4 fachen, speziell mehr als 6 bis 8-fachen Dicke der restlichen durchsichtigen Folie der Abdeckung; bzw.
- einer 1,5 bis 15-fachen, insbesondere 4 bis 12-fachen, speziell 7 bis 10-fachen Dicke der restlichen durchsichtigen Folie der Abdeckung (die z.B. 30 bis 50 µm dick sein kann).

Gemäß einer weiteren Ausführungsform umfasst der verstärkte Folienabschnitt ein anderes Material als die restliche durchsichtige Folie der Abdeckung, insbesondere ein von der Materialnatur her steiferes bzw. knickstabileres Material.

In spezieller Ausführung weist der verstärkte Folienabschnitt eine Transparenz im Infrarotlicht-Wellenlängenbereich und/oder im sichtbaren Lichtwellenlängenbereich auf. Er kann rechteckig ausgebildet sein und sich von einer an eine gegenüberliegende Kante der Abdeckung erstrecken.

Die Halte- bzw. Spanneinrichtung einer sterilen Abdeckung gemäß der vorliegenden Erfindung kann unterschiedlichste Formen annehmen. Als Beispiele seien hier genannt: Ein Band, insbesondere ein Band-Loch-Zug (wie bei Schnürsenkeln an Schuhen); ein Gummizug; ein Klettverschluss; ein Hakenzug oder Hakenverschluss; ein Haft- oder Klebeverschluss. Natürlich kann jedwede Kombination aus mindestens zweien der obigen oder anderen Halte- bzw. Spanneinrichtungen zum Einsatz kommen.

Die Erfindung wird im Weiteren anhand von Ausführungsformen näher erläutert. Sie kann alle hierin beschriebenen Merkmale einzeln oder in jedweder Kombination umfassen. In den beiliegenden Figuren zeigen:
- Figur 1: eine erfindungsgemäße Abdeckung in Gestalt einer Schlauch-Folie; und
- Figur 2: einen verstärkten, vorgefalteten Folienabschnitt einer erfindungsgemäßen Abdeckung.

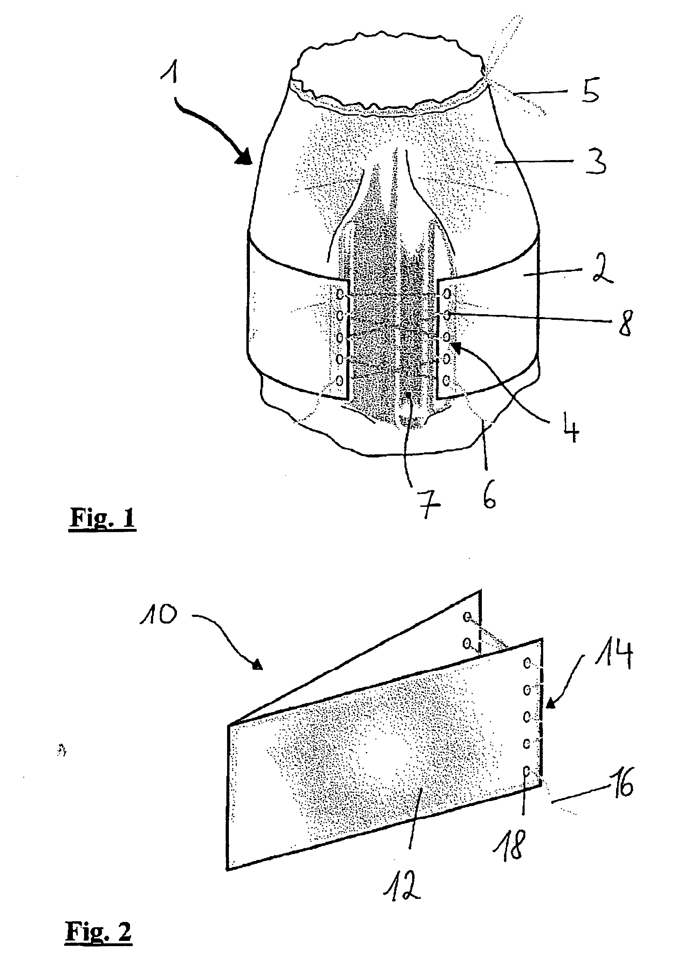

In der Figur 1 ist eine erfindungsgemäße sterile Abdeckung, die schlauchartig ausgebildet ist, insgesamt mit dem Bezugszeichen 1 bezeichnet. Sie umfasst einen ringförmigen verstärkten Abschnitt 2 und einen Restschlauch 3 aus herkömmlichem, dünnem Folienmaterial. Das Folienmaterial in den Abschnitten 2 und 3 ist lichtdurchlässig und/oder infrarotdurchlässig. Der dünnere Folienabschnitt 3 weist an der Oberseite einen Fadenzug 5 auf, mit dem er an dieser Seite verschlossen werden kann. Der demgegenüber materialverdickte Folienabschnitt 2 hat an seiner offenen Seite einen Bandzug 4 mit Löchern 8 an den Endkanten und einem Band 6. Der Bandzug 4 kann wie die Schnürsenkel eines Schuhs eng bzw. straff gezogen und dabei über einen abzudeckenden Gegenstand drapiert werden. Mit dem Bezugszeichen 7 ist noch angedeutet, dass die Folie 3 sich natürlich in dem Bereich, wo der Bandzug 4 sich zusammenzieht, ebenfalls zusammenzieht und faltig wird.

Die erfindungsgemäße Abdeckung 1 kann als C-Bogen-Abdeckung (Drape) verwendet werden, und sie weist eine unsterile Innenseite sowie eine sterile Außenseite auf. Sie kann in dem Sinne "dicht" verschlossen werden, das ihre Teile derart miteinander verbunden sind, dass die Sterilität der Außenseite des Drapes 1 bei unsteriler Innenseite gewährleistet ist (beispielsweise über Schweißnähte, Klebenähte, Klebestellen oder durch großzügige Überlappungen). Das obere Ende des Folienabschnittes 3, das die Form einen Schlauches aufweist, kann beim Bedecken eines C-Bogens von der Bildverstärkerseite des C-Bogens her über den Arm gestülpt bzw. gelegt werden. Dieses Ende hat dann den Bandzug 5, um die Abdeckung 1 am C-Bogen zu befestigen bzw. um das Ende mit dem Bandzug 5 um den Arm herum zu schließen. Es können dabei beispielsweise auch Klebestreifen verwendet werden.

Am unteren Teil ist der verstärkte Folienabschnitt 2 vorgesehen, der durchsichtig ist und eine starre Flexibilität aufweist. Dieser Folienabschnitt 2 kann aufgrund seiner Materialeigenschaften und Stabilität zwar gebogen, aber bei sachgemäßem Gebrauch nicht geknickt werden, so dass er keine Falten wirft. Der Abschnitt 2 besteht aus einem Material, das im optischen und im infraroten Spezialbereich transparent ist, und seine Form ist derart ausgelegt, dass die längeren Seiten dicht mit dem Folienabschnitt 3 verbunden sind. Die kürzeren Seiten, d.h. die Kanten sind flexibel verbunden (oder können flexibel verbunden werden), und zwar in einer solchen Weise, dass sie mit Hilfe der Halte- bzw. Spanneinrichtung, d.h. des Bandzuges 4 aneinander angenähert und fixiert werden können.

Das in Figur 1 dargestellte untere Ende der Abdeckung 1 besteht wieder aus dem Folienabschnitt 3, nämlich der typischen und herkömmlichen Abdeckfolie. Der untere Teil kann geschlossen (beutelartig) oder wiederum auf geeignete Weise dicht verschließbar sein (Schnüre, Klebestreifen). Der Zwischenraum an der Faltstelle 7 ist mit Folie dicht verschlossen. Dieser Abschnitt 7 kann eine Verlängerung der Folie 3 oder auch der verstärkten Folie 2 sein, und ganz allgemein können alle Teile integral ausgestaltet, integral miteinander verbunden, aus einem Stück gefertigt sein, oder einzeln vorliegen und miteinander verbunden bzw. miteinander angeordnet werden.

Die Figur 2 zeigt noch eine Ausführungsform 10 eines verstärkten Folienabschnittes einer erfindungsgemäßen Abdeckung, d.h. lediglich das verstärkte Folienmaterial 12. Auch dieser Abschnitt weist einen Bandzug 14 mit Löchern 18 und einem Band 16 auf, und er hat an der gegenüberliegenden Seite einen Falz, so dass sich der verstärkte Folienabschnitt 10 definiert falten lässt. Solche vorgegebenen Falze oder Faltstellen sind für den Transport und die Lagerung vorteilhaft, weil die Abdeckung so zum Verstauen oder zum Transportieren nicht an Stellen gefaltet wird, die nicht dafür vorgesehen sind. Schädigungen, welche die Funktion beeinträchtigen können, werden verhindert. Es können mehrere Falze vorhanden sein, aber auch "dichte" Scharniermechanismen.

Die erfindungsgemäße Abdeckung eignet sich besonders dazu, außen über ein zylinderförmiges Registrierungs-Kit für C-Bogen-Bildverstärker gezogen zu werden. Die dann zylinderartig angeordnete verstärkte Folie kann eng anliegen und dafür sorgen, dass die Sichtbarkeit von äußeren Reflektorenmarkern nicht beeinträchtigt wird, obwohl die Sterilität gewährleistet ist.

## Patentansprüche

1. Sterile Abdeckung (1), insbesondere für Trackingaufsätze von C-Bogen-Röntgengeräten, mit einer durchsichtigen Folie, **dadurch gekennzeichnet, dass** sie einen verstärkten Folienabschnitt (2) aufweist, sowie eine Halte- bzw. Spanneinrichtung (4) mittels welcher der verstärkte Folienabschnitt an oder über einer Oberfläche straff gezogen werden kann.

2. Sterile Abdeckung nach Anspruch 1, **dadurch gekennzeichnet, dass** sie bzw. zumindest ihr verstärkter Abschnitt als Umgriff oder Umhüllung für den abzudeckenden Gegenstand ausgebildet ist.

3. Sterile Abdeckung nach Anspruch 1 oder 2, **dadurch gekennzeichnet, dass** sie als beutelartige Umhüllung mit einem verstärkten Abschnitt (2) und einem nicht verstärkten Restabschnitt (3) ausgebildet ist.

4. Sterile Abdeckung nach einem der Ansprüche 1 bis 3, **dadurch gekennzeichnet, dass** der verstärkte Folienabschnitt (2) formverstärkt ist, so dass er insbesondere eine glatte Form aufrechterhalten kann.

5. Sterile Abdeckung nach einem der Ansprüche 1 bis 4, **dadurch gekennzeichnet, dass** der verstärkte Folienabschnitt (2) ein verdicktes Material aufweist, insbesondere ein Material mit den folgenden Eigenschaften:
- einer mehr als 1,5-fachen, insbesondere mehr als 3-4 fachen, speziell mehr als 6 bis 8-fachen Dicke einer klinischen, sterilen Abdeckfolie; bzw.
- einer 1,5 bis 15-fachen, insbesondere 4 bis 12-fachen, speziell 7 bis 10-fachen Dicke einer klinischen, sterilen Abdeckfolie.

6. Sterile Abdeckung nach einem der Ansprüche 1 bis 4, **dadurch gekennzeichnet, dass** der verstärkte Folienabschnitt (2) ein verdicktes Material aufweist, insbesondere ein Material mit den folgenden Eigenschaften:
- einer mehr als 1,5-fachen, insbesondere mehr als 3-4 fachen, speziell mehr als 6 bis 8-fachen Dicke der restlichen durchsichtigen Folie der Abdeckung; bzw.
- einer 1,5 bis 15-fachen, insbesondere 4 bis 12-fachen, speziell 7 bis 10-fachen Dicke der restlichen durchsichtigen Folie der Abdeckung.

7. Sterile Abdeckung nach einem der Ansprüche 1 bis 6, **dadurch gekennzeichnet, dass** der verstärkte Folienabschnitt (2) ein anderes Material als die restliche durchsichtige Folie der Abdeckung aufweist, insbesondere ein von der Materialnatur her steiferes bzw. knickstabileres Material.

8. Sterile Abdeckung nach einem der Ansprüche 1 bis 7, **dadurch gekennzeichnet, dass** der verstärkte Folienabschnitt (2) eine Transparenz im Infrarotlicht-Wellenlängenbereich und/oder im sichtbaren Lichtwellenlängenbereich aufweist.

9. Sterile Abdeckung nach einem der Ansprüche 1 bis 8, **dadurch gekennzeichnet, dass** der verstärkte Folienabschnitt (2) rechteckig ausgebildet ist und sich von einer an eine gegenüberliegende Kante der Abdeckung erstreckt.

10. Sterile Abdeckung nach einem der Ansprüche 1 bis 9, **dadurch gekennzeichnet, dass** die Halte- bzw. Spanneinrichtung (4) umfasst:
- einen Bandzug, insbesondere einen Band-Loch-Zug;
- einen Gummizug;
- einen Klettverschluss;
- einen Hakenzug oder Hakenverschluss;
- einen Haft- oder Klebverschluss;
oder eine Kombination aus mindestens zweien der obigen Einrichtungen.

## Claims

1. A sterile drape (1), in particular for tracking attachments of C-arm x-ray apparatus, comprising a transparent film, **characterised in that** it comprises a reinforced film portion (2) and a holding or tensing means (4) by means of which the reinforced film portion can be drawn taut on or over a surface.

2. The sterile drape according to Claim 1, **characterised in that** it or at least its reinforced portion is formed as a wrap-around or sheath for the object to be covered.

3. The sterile drape according to Claim 1 or 2, **characterised in that** it is formed as a pouch-like sheath with a reinforced portion (2) and a non-reinforced remaining portion (3).

4. The sterile drape according to any one of Claims 1 to 3, **characterised in that** the reinforced film portion (2) is reinforced in its shape such that it can in particular maintain a smooth shape.

5. The sterile drape according to any one of Claims 1 to 4, **characterised in that** the reinforced film portion (2) comprises a thickened material, in particular a material having the following properties:
- being more than 1.5 times, in particular more than 3 to 4 times, especially more than 6 to 8 times as thick as a clinical sterile covering film; and/or
- being 1.5 to 15 times, in particular 4 to 12 times, especially 7 to 10 times as thick as a clinical sterile covering film.

6. The sterile drape according to any one of Claims 1 to 4, **characterised in that** the reinforced film portion (2) comprises a thickened material, in particular a material having the following properties:
- being more than 1.5 times, in particular more than 3 to 4 times, especially more than 6 to 8 times as thick as the remaining transparent film of the drape; and/or
- being 1.5 to 15 times, in particular 4 to 12 times, especially 7 to 10 times as thick as the remaining transparent film of the drape.

7. The sterile drape according to any one of Claims 1 to 6, **characterised in that** the reinforced film portion (2) comprises a different material to the remaining transparent film of the drape, in particular a material which by its material nature is more rigid and/or more stable against folding.

8. The sterile drape according to any one of Claims 1 to 7, **characterised in that** the reinforced film portion (2) exhibits a transparency in the infrared wavelength range and/or in the visible wavelength range.

9. The sterile drape according to any one of Claims 1 to 8, **characterised in that** the reinforced film portion (2) is formed to be rectangular and extends from one edge of the drape to an opposite edge of the drape.

10. The sterile drape according to any one of Claims 1 to 9, **characterised in that** the holding or tensing means (4) comprises:
- a belt tie, in particular a belt-hole tie;
- a rubber tie;
- a Velcro fastening;
- a hooked tie or hooked fastening;
- an adhesive or bonded fastening;
or a combination of at least two of the above means.

## Revendications

1. Couverture stérile (1), en particulier pour un accessoire de suivi pour un appareil de radiographie sur arceau, avec un film transparent, **caractérisée en ce qu'**elle comporte une section de film renforcée (2) ainsi qu'un dispositif de maintien ou de serrage (4) au moyen duquel la section de film renforcée peut être tendue au niveau de ou par-dessus une surface.

2. Couverture stérile selon la revendication 1, **caractérisée en ce qu'**elle ou du moins sa section renforcée est conçue comme un élément de serrage ou comme une enveloppe pour l'objet à couvrir.

3. Couverture stérile selon la revendication 1 ou 2, **caractérisée en ce qu'**elle est conçue comme une enveloppe en forme de sac avec une section renforcée (2) et une section restante non renforcée (3).

4. Couverture stérile selon l'une quelconque des revendications 1 à 3, **caractérisée en ce que** la section de film renforcée (2) est renforcée en termes de forme, de manière à ce qu'elle puisse maintenir en particulier une forme lisse.

5. Couverture stérile selon l'une quelconque des revendications 1 à 4, **caractérisée en ce que** la section de film renforcée (2) comporte un matériau épaissi, en particulier un matériau ayant les propriétés suivantes :
- une épaisseur plus de 1,5 fois, en particulier plus de 3 à 4 fois, spécifiquement plus de 6 à 8 fois plus épaisse qu'un film de couverture stérile clinique, respectivement
- une épaisseur 1,5 à 15 fois, en particulier 4 à 12 fois, spécifiquement 7 à 10 fois plus épaisse qu'un film de couverture stérile clinique.

6. Couverture stérile selon l'une quelconque des revendications 1 à 4, **caractérisée en ce que** la section de film renforcée (2) comporte un matériau épaissi, en particulier un matériau ayant les propriétés suivantes :
- une épaisseur plus de 1,5 fois, en particulier plus de 3 à 4 fois, spécifiquement plus de 6 à 8 fois plus épaisse que le reste du film transparent de la couverture, respectivement
- une épaisseur 1,5 à 15 fois, en particulier 4 à 12 fois, spécifiquement 7 à 10 fois plus épaisse que le reste du film transparent de la couverture.

7. Couverture stérile selon l'une quelconque des revendications 1 à 6, **caractérisée en ce que** la section de film renforcée (2) comporte un autre matériau que le reste du film transparent de la couverture, en particulier un matériau plus rigide ou plus résistant aux pliures.

8. Couverture stérile selon l'une quelconque des revendications 1 à 7, **caractérisée en ce que** la section de film renforcée (2) comporte une transparence sur la plage de longueur d'onde de la lumière infrarouge et/ou sur la plage de longueur d'onde de la lumière visible.

9. Couverture stérile selon l'une quelconque des revendications 1 à 8, **caractérisée en ce que** la section de film renforcée (2) comporte une forme rectangulaire et s'étend d'un bord à un bord opposé de la couverture.

10. Couverture stérile selon l'une quelconque des revendications 1 à 9, **caractérisée en ce que** le dispositif de maintien ou de serrage (4) comprend :
- un cordon de serrage, en particulier un cordon de serrage avec stoppeur à trous ;
- un cordon élastique
- une fermeture de type velcro®
- un cordon ou une fermeture à crochets ;
- une fermeture adhésive ;
ou une combinaison d'au moins deux de ces dispositifs.
